**Europäisches Patentamt**

**European Patent Office**

**Office européen des brevets**

(19)

(11) Veröffentlichungsnummer: **0 290 538**
**B1**

(12) **EUROPÄISCHE PATENTSCHRIFT**

(45) Veröffentlichungstag der Patentschrift: **11.07.90**

(21) Anmeldenummer: **87907596.8**

(22) Anmeldetag: **13.11.87**

(86) Internationale Anmeldenummer:
**PCT/EP87/00702**

(87) Internationale Veröffentlichungsnummer:
**WO 88/03416 19.05.88 Gazette 88/11**

(51) Int. Cl.⁵: **A 61 L 11/00, A 61 M 5/32**

(54) **VORRICHTUNG ZUR HYGIENISCHEN ABFALLAUFNAHME IN KRANKENHÄUSERN, LABORATORIEN UND ÄHNLICHEN INSTITUTEN.**

(30) Priorität: **14.11.86 DE 8630535 u**

(43) Veröffentlichungstag der Anmeldung:
**17.11.88 Patentblatt 88/46**

(45) Bekanntmachung des Hinweises auf die
Patenterteilung:
**11.07.90 Patentblatt 90/28**

(84) Benannte Vertragsstaaten:
**AT BE CH DE FR GB IT LI NL SE**

(56) Entgegenhaltungen:
**DE-A-3 317 300**
**US-A-4 552 720**

(73) Patentinhaber: **Löbbert, Johannes**
**Lindenstrasse 22**
**D-4405 Nottuln-Appelhülsen (DE)**

(72) Erfinder: **Löbbert, Johannes**
**Lindenstrasse 22**
**D-4405 Nottuln-Appelhülsen (DE)**

(74) Vertreter: **Hoffmeister, Helmut, Dr. Dipl.-Phys.**
**Patentanwalt Goldstrasse 36**
**D-4400 Münster (DE)**

Courier Press, Leamington Spa, England.

EP 0 290 538 B1

## Beschreibung

Die Erfindung betrifft eine Vorrichtung zur hygienischen Aufnahme von infektiösen oder sonstigen kontaminierten Abfällen, beispielsweise in Krankenhäusern, Arztpraxen, Laboratorien und ähnlichen Instituten, bei welcher Vorrichtung die Abfälle von einem Behälter, der von einer Haltevorrichtung getragen ist, aufgenommen werden und der Behälter mit einem bereitgehaltenen Deckel nach Füllung hermetisch verschließbar ist.

Medizinische Abfälle, wie sie in Krankenhäusern, Arztpraxen, Laboratorien und ähnlichen Instituten anfallen, sind teilweise hoch-infektiös. Derartige Abfälle müssen gesammelt, gelagert und beseitigt werden, ohne daß Gefahr besteht, daß sie anschließend während des Sammelns, Lagern oder Beseitigens weitere Kontaminationsgefahren auslösen. In den genannten Instituten wird der Abfall bislang im allgemeinen in aufgespannte Plastiksäcke oder manuell zu bedienende Kunststoffbehälter eingeworfen, die nach Befüllung manuell, gegebenenfalls mit einem bereitgehaltenen Deckel nach Füllung hermetisch verschlossen und anschließend zur Verbrennung transportiert werden.

Es hat sich gezeigt, dab diese Art der Entsorgung erhebliche Risiken in sich bringt, und zwar einerseits durch die manuellen Tätigkeien und andererseits durch manuelles Öffnen und Schließen der Kunststoffbehälter, was sowohl während der Aufnahme als auch während des Verschließens zu Turbulenzen der über dem Behalter stehenden Luftmenge fßhrt, wobei Krankheitserreger in die Umgebung getragen werden können. Sowohl bei der Verdrängungs- als auch bei der Öffnungsbewegung kommt es damit zu Aerosol-Transporten von Giften oder pathogenen Krankheitserregern aus dem Bereiche des Behälterinneren.

Es stellt sich demnach die Aufgabe, die Nachteile des Standes der Technik zu beseitigen und eine Vorrichtung zur hygienischen Abfallaufnahme anzugeben, bei der manuelle Kontakte mit dem Abfallbehältnis weitestgehend vermieden werden und eine ungewollte Ausbreitung der Krankheitserreger praktisch völlig verhindert ist, wobei auf an sich bekannte Behälter zurückgegriffen werden kann. Das hermetische Verschließen soll ohne jede Kontaminationsmöglichkeit bewerkstelligt werden. Infektionsgefahr durch den Lufttransport von Mikroorganismen oder toxischen Stäuben soll weitestgehend vermieden werden.

Diese Aufgabe wird gelöst bei einer Vorrichtung der eingangs genannten Art, die dadurch gekennzeichnet ist, daß der Deckel von einem an der Haltevorrichtung schwenkbeweglich gehaltenen Deckelhalter freigebbar getragen ist, der von einem motorischen Antrieb bewegbar ist und das Öffnen und Schließen des Behälters besorgt, wobei der Deckel während des Schließens in der Befüllungsphase lose auf den Behälter aufgelegt ist, und dadurch, daß mit Hilfe eines weiteren, mit der Vorrichtung verbundenen Antriebes der Deckelhalter mit dem Deckel auf den Behälter fest verschließend aufpreßbar ist und nach dem Verbinden des Deckels mit dem Behälter den Deckel freigibt.

Mit der vorgenannten Vorrichtung werden demnach zwei unterschiedliche, aber für die Aufgabenlösung notwendige Tätigkeiten durchgeführt: zum einen wird der Deckelhalter mit dem später aufzubringenden Deckel motorisch angetrieben so bewegt, daß quasi im Zeitlupentempo eine Bewegung erfolgt, bei der erfahrungsgemäß jede Turbulenz-Erzeugung ausgeschlossen wird. Diese Deckelhalter-Bewegung wird jedesmal dann durchgeführt, wenn erneut Abfall in den Behälter eingeworfen werden soll. Zum anderen wird der später hermetisch mit dem Behälter zu verbindende Deckel im Deckelhalter bereitgehalten, während der Befüllungsphase immer wieder lose auf den Behälter aufgelegt und dann, wenn der Behälter gefüllt ist, mit Hilfe des Deckelhalters und der Haltevorrichtung auf den Behälter fest aufgepreßt, so daß bei entsprechender Ausbildung von Deckel und Behälter dieser beispielsweise am Deckel rundherum einschnappt und gehalten ist und dadurch ein sicherer Verschluß bewirkt wird. Diese Tätigkeit vollzieht sich beir geschlossener Vorrichtung und vorzugsweise vollautomatisch.

Beispielsweise ist möglich, daß der Behälter, wie an sich bekannt, mit einem Wulstrand umgeben ist. In diesem Falle ist es vorteilhaft, wenn die Haltevorrichtung ein offenes, mit einem Rand unter den Wulstrand des Behälters greifendes Aufnahmegefäß umfaßt, das den Behälter trägt und ihn auch bei Erfordernis von erheblichen Kräften sicher tragen kann.

Der Behälter kann für sich allein, vorzugsweise jedoch mit dem Aufnahmegefäß auf- und abbewegbar sein. Hierzu kann unterhalb des Aufnahmegefäßes ein hydraulischer Stempel, eine Schraubspindel-Vorrichtung oder ein Schren-Schemel vorgesehen sein; dies sind Vorrichtungen, wie sie dem Fachmann der Fördertechnik geläufig sind und wie sie sich als construcktive Lösungen anbieten.

Um eine einwandfreie Halterung des Behälters bei gleichzeitiger, leichter Abnehmbarkeit zu gewärkleisten, ist der Boden des Aufnahmesgefäßes formschlüssig gehalten und ruht auf der Platte, die mit Hilfe eines Antriebes auf- und abbeweglich ist.

Der Deckelhalter ist mit federnden Klammern, die auch aus Gummi ausgeführt sein können, ausgerüstet. In diese Klammern kann de Deckel einfach eingelegt werden. Später wird er dann automatisch entnommen. Hierfür ist die Klammerkraft so eingestellt, daß der auf den Behälter fest aufgesetzte Deckel sich aus den Klammern löst.

Der Deckelhalter wird vorzugsweise mit Hilfe eines motorischen Antriebes verschwenkt, wobei über ein Zwischengetriebe dafür gesorgt wird, daß die Schwenkbewegung sich in einem äußerst langsamen Tempo vollzieht. Hierzu kann ein Schubtrieb vorgesehen sein.

Sämtliche Vorrichtungen können auch unabhängig von einer elektrischen Stromversorgung ausgerüstet sein. Beispielsweise kann das Heben und Senken des Deckels auch über eine über Pedal angetriebene Hydraulikpumpe geschehen. Ebenso ist es möglich, daß die Haltevorrichtung mit einer beweglichen, lösbaren Klammervorrichtung ausgestattet ist, die in kompatible Teile des Deckelhalters eingreift und mit der der Deckelhalter gegen die Haltevorrichtung anziehbar ist, was beispielsweise ebenfalls über ein Pedal geschehen kann. Jedenfalls ist möglich, den Grundgedanken der Erfindung auch mit Vorrichtungsteilen auszuführen, die von einer externen Stromsversorgung unabhängig sind. Grundsätzlich wird aber einer technischen Lösung der Vorzug gegeben, bei der eine Vorrichtung geschaffen wird, die durch den Einsatz elektrischer Energie den Menschen entlastet und durch nicht-körperlich ausgelöste Steuerungen manuelle Tätigkeiten, insbesondere das Andrücken von schwergängigen und schwer steuerbaren Pedalen erübrigt und damit unkontrollierte Bewegungen des Deckels und damit Kontaminationen ausschließt.

Ausführungsbeispiele der Erfindung werden in der Zeichnung dargestellt. Die Figuren der Zeichnung zeigen im einzelnen:

Figur 1 in schematischer Schnittansicht eine Vorrichtung gemäß einer ersten Ausführungsform;

Figur 2 eine zweite Ausführungsform;

Figur 3 ein Detail der Deckelausgestaltung.

Die Vorrichtungf zur hygienischen Aufnahme von infektiösen und sonstigen kontaminierten Abfällen, beispielsweise in Krankenhäusern, Arzpraxen, Laboratorien und ähnlichen Instituten hat eine solche Höhe, daß der Zugang zu einem in der Vorrichtung befindlichen Behälter einfach ist und keine besonderen Schwierigkeiten bereitet. Die Vorrichtung kann demnach aufgestellt, jedoch auch in den Boden eingesenkt sein. Die Vorrichtung ist mit einem entnehmbaren Behälter 18 ausgerüstet, der beispielsweise die Gestalt eines zylindrischen Kegelstumpfes hat. Der Behälter besteht aus einem Kunststoffmaterial, beispielsweise Polyethylen. Der Behälter ist von einer Haltevorrichtung 1 getragen, die ein geschlossenes Gehäuse 10 mit einem Boden 2 aufweist. Äußerlich hat die Haltevorrichtung beispielsweise die Form eines Zylinders oder eines polygonalen Prismas oder dergleichen. Im Inneren des Gehäuses 10 ist ein Aufnahmegefäß 7 angeordnet, das beispielsweise aus einem geschlossenen Kunststoff- oder Blechzylinder besteht, wobei auch hier bei der Gestaltung hygienische Erfordernisse im Vordergrund stehen. Das Aufnahmegefäß 7 endet nach oben mit einem Rand, der unter einen Wulstrand 25 greift, der zum Behälter 18 gehört und dessen oberen Abschluß bildet. Dementsprechend hängt der Behälter freischwebend innerhalb des Aufnahmesgefäßes 7.

Das Aufnahmegefäß 7 kann entweder fest mit der übrigen Haltevorrichtung verbunden sein. Im vorliegenden Falle ist jedoch eine Konstruction gewählt worden, bei der das den Behälter 18 tragende Aufnahmegefäß 7 auf- und abbeweglich ist. Hierzu ist das Aufnahmegefäß 7 innerhalb eines Kragens 34 geführt, der vom Außenmantel her vom Gehäuse 10 gebildet wird. Unterhalb des Aufnahmegegäßes 7 ist dieses mit einer in das Innere des Gefäßes reichenden Einwölbung 20 versehen, dies es gestattet, daß das Gefäß 7 formschlüssig auf eine Platte 6 aufgesetzt wird. Die Platte 6 ist in Richtung einer Achse 23 auf- und abbeweglich, und zwar durch einen hier schematisch dargestellten Antrieb, der aus einer Spindel 5 und einem Antriebsmotor 4 besteht. Der Motor 4 kann durch einen am Gehäuse angbrachten Schalter (nicht dargestellt) in Bewegung gesetzt werden, wobei durch eine entsprechende, automatische Abstellung eine bestimmte Höhe oder Tiefe nicht über- oder unterschritten wird. Wie bereits angedeutet, kann anstelle des Spindelantriebes 4, 5 auch eine andere Höhenverstellung vorgenommen werden, beispielsweise durch hydraulische oder pneumatische Kolben-Zylinder-Anordnungen, durch einen spindelgetriebenen, höhenverstellbaren Schemel oder dergleichen. Der Boden 8 des Aufnahmegefäßes 7 kann demnach zusammen mit dem Behälter auf- und abbewegt werden.

Weiterhin wesentlich für die Erfindung ist ein Deckelhalter 14, der schwenkbeweglich an der Haltevorrichtung 1 angelenkt ist. Hierzu ist ein Schwenkgelenk 12 vorgesehen, das es ermöglicht, daß der Deckelhalter 14 in die durch den Pfeil 24 angedeuteten Richtungen beweglich ist. Das Schwenken geschieht nicht durch eine pedalbetriebene oder manuelle Vorrichtung, sondern mit Hilfe eines Antriebsmotors 3, der beispielsweise über einen Exzentertrieb und eine Stange 9, insgesamt Schubtrieb genannt, an einem Drehgelenk 13 angreift und damit das Öffnen und Schließen mit Hilfe eines taktsteuernden Schalters durchführt. Hierzu ist im allgemeinen ein Fußschalter vorgesehen. Es ist jedoch auch möglich, einen Näherungsschalter, akustisch steuerbaren Schalter oder dergleichen vorzusehen. Auch derartige Schaltelemente sind dem Fachmann an sich bekannt. Das Öffnen und Schließen des Deckels ist dabei in seiner Geschwindigkeit so verlangsamt, daß es quasi im Zeitlupentempo erfolgt und zuverlässig ausschließt, daß gefährliche Luft-Turbulenzen im Bereich zwischen Deckelhaltevorrichtung und Behälteröffnung entstehen.

Unterhalb des Deckelhalters 14 ist in einer Klammervorrichtung, schematisch dargestellt durch die Klammer 17, ein Behälterdeckel 19 entnehmbar gehalten. Solange der Behälter 18 nicht gefüllt ist, öffnet und schließt sich der Deckelhalter 14 zusammen mit dem Deckel 19 und legt den Deckel 19 dicht, jedoch relativ lose auf den Behälter auf. Jedenfalls wird beim Öffnen des Deckelhalters 19 der Deckel immer wieder mitgenommen. Ist dagegen der Behälter 18 gefüllt und muß abtransportiert werden, so wird der Deckelhalter 14 in seiner waagerrechten Position arretiert. Der Antriebsmotor 4 wird in Gang gesetzt und hebt die Haltevorrichtung mit dem Behälter gegen den arretierten Deckelhalter 14. Dabei wird der Deckel auf den Behälter aufgepreßt. Es kommt zu einem Überstülpen des Deckelrandes 33 auf den Wulstrand 25 des Behälters, wobei im allgemeinen ein hörbares "Klack"-Geräusch ertönt. Nunmehr ist der Behälter hermetisch verschlossen und nur noch mit erheblicher Kraft überhaupt vom Behälter 18 lösbar. Wird jetzt der Deckelhalter 14 wieder nach oben geschwenkt, so löst sich der Deckel aus der klammernden Halterung und wird freigegeben. Anschließend wird der Behälter manuell oder mit Hilfe einer Vakuum-Tragevorrichtung entnommen. In den Deckelhalter 14 kann ein neuer Deckel eingelegt werden.

Für eine Verwendung innerhalb der vorgeschriebenen Vorrichtung eignen sich Behälter mit Deckel, die endgültig und sicher verschließbar sind. Es handelt sich demnach insebesondere um zylindrische Einwegbehälter aus Kunststoff oder Metall zum endgültigen Vernichten oder Ablagern von Inhaltsgütern. Unberechtigte Zugriffe und Inhaltsverluste müssen ausgeschlossen werden. Entsprechend schwer zu öffnende Schnappverschüsse sind daher technisch zu realisieren. Der Wulstrand 25 am Behälter 18 und ein entsprechend ausgebildeter Deckelrand 33 werden angewendet, wobei das Verschließen eine relativ lange axiale Bewegung unte Kraftaufwendung in Richtung der Achse 23 erforderlich.

Die Ausführung des Wulstrandes 25 des Behälters 18 kann auch so erfolgen, daß das Aufnahmegefäß 7 an seinem Oberrand mit bestimmten Passungen versehen ist, z.B. aus Nut und Feder. Hierdurch wird gewährleistet, daß nur bestimmte, zugelassene Behälter verwendet werden können, die zudem nur in einer bestimmten Position eingesetzt werden können. Daraus geht hervor, daß die Haltevorrichtung 1 in ihren aufnehmenden und/oder unterstützenden Teilen eineindeutig einem bestimmten Bahältertyp zugeordnet ist.

Der Deckelhalter 14 ist mit relativ weit heruntergezogenen, schürzenartigen Rändern 15 versehen, die außerdem noch mit wulstartigen Anlagekanten 15 ausgestattet sein können, die mit Federn 16 angepreßt werden. Hierdurch ergibt sich der Vorteil, daß sich der Deckel fest an die Außenseite 11 des Gehäuses 1 anlegt.

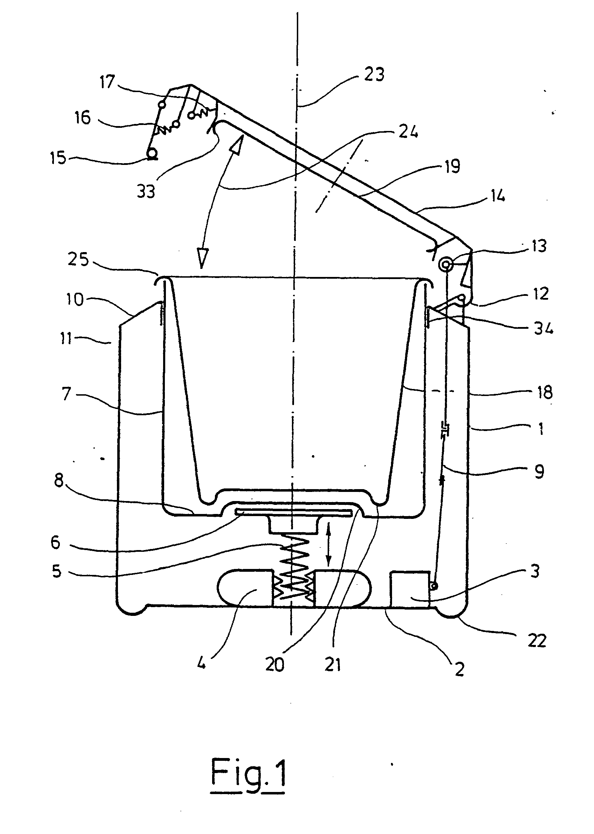

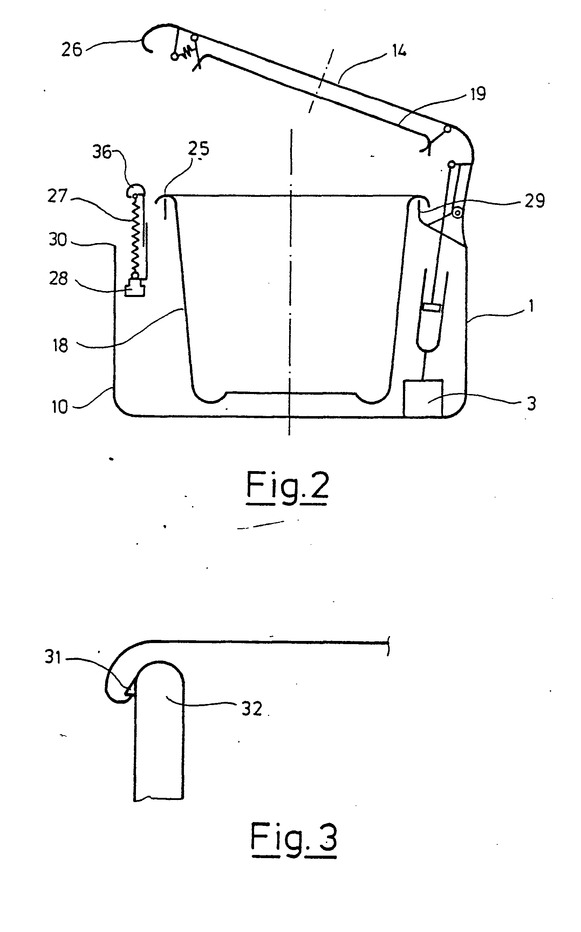

In Figur 2 ist eine etwas vereinfachte Vorrichtung dargestellt. Auch hier findet sich eine Haltevorrichtung 1, in die ein Behälter 18 eingehängt ist. Der Behälter ist jedoch nicht auf- und abbeweglich. Der Deckelhalter 14 mit dem darin gehaltenen Deckel 19 wird wiederum über einen Antriebsmotor 3 motorisch angetrieben. Hierfür ist eine ähnliche Technik vorgesehen, wie dies im Ausführungsbeispiel gemäß Figur 1 dargestellt ist. Der Wulstrand 25 des Behälters 18 wird getragen von einer Unterfassung 29.

In einer Abschrägung 30 des Gehäuses 10 ist eine Klammervorrichtung 27 vorgesehen, die einen nach unten gegen Federkraft ziehenden Haken 36 aufweist. Das Ziehen kann durch einen Knauf 28 geschehen. Der Knauf kann jedoch auch durch eine Pedalvorrichtung oder dergleichen ersetzt sein. Wird der Deckelhalter 14 aufgelegt, so kann dann, wenn das Aufpressen des Deckels 19 erforderlich ist, der Knauf 18 gegen die Federkraft nach unten gezogen werden und preßt dabei den Deckelhalter 14 mit dem Deckel 19 auf den Behälter 18, wobei sich der Deckel mit diesem verbindet. Nach dem Loslassen und Ent-Arretieren des Hakens 36 ist der Deckelhalter wiederfreigegeben. Nach dem Öffnen kann ein neuer Deckel 19 eingelegt werden. Der Deckelhalter 14 ist entsprechend kompatibel ausgerüstet, so daß der Haken 36 in ihn eingreifen kann.

Figur 3 zeigt ein Detail einer gegenüber Figur 2 etwas abgewandelten Lösung. Der Deckelhalter 14 besitzt hier eine Klinke 26, in die eine Rastklinke 31 einer verstellbaren Vorrichtung 32 einrasten kann. Beim Zurückziehen der Rastklinke 31 mit Hilfe der Vorrichtung 32 wird das ganze wieder gelöst.

Auch hier erfolgt das Verschließen des Behälters 18 mit dem Deckel 19 durch das Anziehen des Rastmittels im eingeklinkten Zustand. Die axiale Beweglichkeit des Deckelhalters 14 wird durch eine entsprechende Beweglichkeit im Schwengkgelenk ermöglicht. Der Behälter wird an mindestens drei Stellen seines Wulstrandes unterfaßt (bei 29).

Es sei noch angemerkt, daß der Deckelhalter 14 auch mit einer den Deckel nach unten drückbaren weiteren Drückvorrichtung (nicht dargestellt) versehen sein kann, die den radial geführten, aber axial beweglichen Behälterdeckel auf den Behälter 18 zu bewegt. Hierdurch kann gegebenenfalls noch eines gleichmäßigere Anpressung des Deckels auf den Behälterrand erfolgen.

Der Öffnungswinkel des Deckelhalters muß so bemessen sein, daß Inhaltsgüter einwandfrei eingeworfen werden können. Im allgemeinen steht daher das Schwenkgelenk 12 hinter dem äußeren Rand der Haltevorrichtung 1 zurück.

Für die Steuerung der verschiedenen Antriebsbewegungen werden vorzugsweise Fußschalter oder berührungslose Schalter verwendet, die in der Zeichnung nicht dargestellt sind, da sie dem Fachmann allgemein bekannt sind.

**Patentansprüche**

1. Vorrichtung zur hygienischen Aufnahme von infektiösen und sonstigen kontaminierten Abfällen, beispielsweise in Krankenhäusern, Arztpraxen, Laboratorien und ähnlichen Instituten, bei welcher Vorrichtung die Abfälle von einem Behälter (18), der von einer Haltevorrichtung (1) getragen ist, aufgenommen werden und der Behälter (18) mit einem bereitgehaltenen Deckel (19) nach Füllung hermetisch verschließbar ist, dadurch gekennzeichnet, daß die Deckel (19) von einem an der Haltevorrichtung (1) schwenkbeweglich gehaltenen Deckelhalter (14) freigebbar getragen ist, der von einem motorischen Antrieb (3) bewegbar ist und das Öffnen und Schließen des Behälters (18) besorgt, wobei der Deckel (19) während des Schließens in der Befüllungsphase lose auf den Behälter (18) aufgelegt ist, und daß mit Hilfe eines weiteren, mit der Vorrichtung (1) verbundenen Antriebes (4, 5; 27, 28) der Deckelhalter (14) mit dem Deckel (19) auf den Bahälter (18) fest verschließend aufpreßbar ist und nach dem Verbinden des Deckels (19) mit dem Behälter (18) den Deckel (19) freigibt.

2. Vorrichtung nach Anspruch 1, bei der der zu verschließende Behälter mit einem Wulstrand (25) umgeben ist, dadurch gekennzeichnet, daß die Haltevorrichtung (1) ein offenes, mit einem Rand unter den Wulstrand (25) des Behälters greifendes Aufnahmegefäß (7) umfaßt.

3. Vorrichtung nach Anspruch 2, dadurch gekennzeichnet, daß das Aufnahmegefäß (7) mit dem Behälter (18) auf- und abbewegbar ist.

4. Vorrichtung nach Anspruch 3, dadurch gekennzeichnet, daß der Boden (8) des Aufnahmegefäßes (7) auf einer Platte (6) ruht, die mit Hilfe eines Antriebs (4) auf- und abbeweglich ist.

5. Vorrichtung nach Anspruch 4, dadurch gekennzeichnet, daß der Boden (8) des Aufnahmegefäßes (7) formschlüssig gehalten auf der Platte (6) ruht.

6. Vorrichtung nach Anspruch 1, dadurch gekennzeichnet, daß der Deckelhalter (14) mit federnden Klammern (17) zum Halten und Lösen des Deckels (19) ausgerüstet ist.

7. Vorrichtung nach Anspruch 1, dadurch gekennzeichnet, daß der Deckelhalter (14) mit Hilfe eines im Bereich eines Schwenkgelenkes (12) angreifenden Schubtriebes (3, 9), der mit einem motorischen Antrieb (3) versehen ist, verschwenkbar ist.

8. Vorrichtung nach Anspruch 1, dadurch gekennzeichnet, daß die Haltevorrichtung (1) mit einer beweglichen, lösbaren Klammervorrichtung (27) ausgestattet ist, die in kompatible Teile des Deckelhalters eingreift und mit der der Deckelhalter (14) gegen die Haltevorrichtung (1) anziebar ist.

9. Vorrichtung nach Anspruch 4 und 5, dadurch gekennzeichnet, daß der Antrieb (3) für die Schwenkbewegung des Deckelhalters (14) und der Antriebsmotor (4) für die Auf- und Abbewegung des Aufnahmegefäßes (7) durch getrennte, auseinanderliegend an der Haltevorrichtung angebrachte Schalter zu betätigen sind.

10. Vorrichtung nach Anspruch 1, dadurch gekennzeichnet, daß der Deckelhalter (14) eine Aufdrückvorrichtung umfaßt, die den Deckel (19) in aufliegendem Zustand auf den Behälter drückt.

11. Vorrichtung nach Anspruch 2, dadurch gekennzeichnet, daß die Haltevorrichtung (1) in ihren Aufnehmenden und/oder unterstützenden Teilen eindeutig einem bestimmten Behältertyp zugeordnet ist.

## Revendications

1. Dispositif pour la collecte hygiénique de déchets infectieux ou d'autres déchets contaminés, par exemple dans des hôpitaux, des cabinets médicaux, des larboratoires ou établisshements similaires, dans lequel les déchets sont collectés dans un conteneur (18) qui est porté par un dispositif de retenue (1) et le conteneur (18) peut, une fois rempli, être fermé hermétiquement par un couvercle (19) disponible, caractérisé en ce que le courvecle (19) est porté de manière à pouvoir être libéré par un support de couvercle (14) qui est maintenu pivotant sur le dispositif de retenue (1), peut être déplacé par un dispositif de commande à moteur (3) et provoque l'ouverture et la fermeture du conteneur (18), le couvercle (19) étant posé sur le conteneur (18) de manière lâche pendant la fermeture dans la phase de remplissage, et en ce qu'au moyen d'un autre dispositif de commande (4, 5; 27, 28) relié au dispositif, le support de couvercle (14) peut être pressé avec le couvercle (19) sur le conteneur (18) de manière à réaliser une fermeture hermétique et libère le couvercle (19) une fois qu'il est relié au conteneur (18).

2. Dispositif selon la revendication 1 dans lequel le conteneur à fermer est entouré par un rebord (25), caractérisé en ce que le dispositif de retenue (1) comprend une cuve de réception (7) ouverte s'engageant par un bord sous le rebord (25) du conteneur.

3. Dispositif selon la revendication 2, caractérisé en ce que la cuve de réception (7) peut être levée et abaissée avec le conteneur (18).

4. Dispositif selon la revendication 3, caractérisé en ce que le fond (8) de la cuve de réception (7) repose sur une plaque (6) qui peut être levée et abaissée à l'aide d'un dispositif de commande (4).

5. Dispositif selon la revendication 4, caractérisé en ce que le fond (8) de la cuve de réception (7) repose sur la plaque (6) ou ètant maintenu par complémentarité de formes.

6. Dispositif selon la revendication 1, caractérisé en ce que le support de couvercle (14) est équipé de pinces élastiques (17) pour retenir et libérer le couvercle (19).

7. Dispositif selon la revendication 1, caractérisé en ce que le support de couvercle (14) peut pivoter à l'aide d'un pignon coulissant (3, 9) qui agit dans la zone de l'axe de pivotement (12) et est muni d'un dispositif de commande à moteur (3).

8. Dispositif selon la revendication 1, caractérisé en ce que le dispositif de retenue (1) est équipé d'un dispositif à pinces (27) mobile et amovible qui s'engage dans des pièces compatibles du support de couvercle et par lequel le support de couvercle (14) peut être tiré vers le dispositif de retenue (1).

9. Dispositif selon les revendications 4 et 5, caractérisé en ce que le dispositif de commande (3) pour le pivotement du support de couvercle (14) et le moteur d'entraînement (4) pour le levage et l'abaissement de la cuve de réception (7) peuvent être actionnés par des commutateurs séparés rapportés sur le dispositif de retenue en étant écartés l'un de l'autre.

10. Dispositif selon la revendication 1, caractérisé en ce que le support de couvercle (14) comprend un dispositif de pression qui presse le couvercle (19) une fois posé sur le conteneur.

11. Dispositif selon la revendication 2, caractérisé en ce que le dispositif de retenue (1) est clairement associé par ses pièces qui recoivent et/ou soutiennent à un certain type de conteneur.

**Claims**

1. A device for the hygienic disposal of infectious and otherwise contaminated waste, for example in hospitals, doctors' surgeries, laboratories and similar institutes, in the case of which device the waste is received by a container (18), which is held by a holding device (1), and the container (18) may be hermetically sealed after filling with a lid (19) provided, characterised in that the lid (19) is so supported that it may be released by a lid holder (14), which is pivotably held on the holding device (1) and movable by a motor drive (3), and which ensures opening and closing of the container (18), the lid (19) being laid loosely on the containing (18) when closed in the filling phase, and in that, by means of a further drive (4, 5; 27, 28) connected to the device (1), the lid holder (14) may be pressed with the lid (19) on to the container (18) so as firmly to seal said container (18), and after connecting of the lid (19) to the container (18) the lid holder (14) releases the lid (19).

2. Device according to claim 1, wherein the container to be sealed is surrounded by a rim (25), characterised in that the holding device (1) incorporates an open receptacle (7) which engages with an edge under the rim (25) of the container.

3. Device according to claim 2, characterised in that the receptacle (7) can be moved up and down with the container (18).

4. Device according to claim 3, characterised in that the bottom (8) of the receptacle (7) rests on a disc (6), which may be moved up and down by means of a drive (4).

5. Device according to claim 4, characterised in that the bottom (8) of the receptacle (7) rests, held with positive locking, on the disc (6).

6. Device according to claim 1, characterised in that the lid holder (14) is equipped with sprung clips (17) for holding and releasing the lid (19).

7. Device according to claim 1, characterised in that the lid holder (14) is pivotable by means of a sliding gear (3, 9) provided with a motor drive (3) and engaging in the region of a pivot joint (12).

8. Device according to claim 1, characterised in that the holding device (1) is equipped with a mobile, detachable clip device (27), which engages in compatible parts of the lid holder and with which the lid holder (14) may be secured against the holding device (1).

9. Device according to claims 4 and 5, characterised in that the drive (3) for the pivoting of the lid holder (14) and the drive motor (4) for the up and down movement of the receptacle (7) are to be actuated by separate switches mounted apart on the holding device.

10. Device according to claim 1, characterised in that the lid holder (14) comprises a pressure device, which presses the lid (19) in the open position on to the container.

11. Device according to claim 2, characterised in that the holding device (1) is clearly associated in its receiving and/or supporting parts with a particular type of container.

Fig.1

Fig.2

Fig.3

2